# EUROPEAN PATENT SPECIFICATION

(11) **EP 2 314 238 B1**
(45) Date of publication and mention of the grant of the patent: **30.01.2019**
(21) Application number: 10764064.1
(22) Date of filing: 05.02.2010
(51) Int. Cl.: A61B 17/56, A61B 17/04, A61B 90/92

(54) **FIXATION DEVICE FOR SUTURING AND RESTORING A TEMPOROMANDIBULAR JOINT DISC**
FIXIERUNGSVORRICHTUNG ZUM NÄHEN UND WIEDERHERSTELLEN EINES KIEFERGELENK-DISKUS
DISPOSITIF POUR SUTURE ET RESTAURATION D'UN DISQUE D'ARTICULATION TEMPORO-MANDIBULAIRE

(30) Priority: 14.04.2009 CN 200910049274
(43) Date of publication of application: 27.04.2011
(73) Proprietor: Shanghai Ninth People's Hospital Affiliated To Shanghai Jiao Tong University School Of Medicine, Huangpu, Shanghai 200011 (CN)
(72) Inventor: YANG, Chi, Huangpu Shanghai 200011 (CN); ZHANG, Shanyong, Huangpu Shanghai 200011 (CN); ZHANG, Zhiyuan, Huangpu District Shanghai 200011 (CN); CHEN, Minjie, Huangpu Shanghai 200011 (CN); CAI, Xieyi, Huangpu Shanghai 200011 (CN); ZHANG, Jinning, Huangpu Shanghai 200011 (CN); LIU, Xiuming, Huangpu District Shanghai 200011 (CN)
(74) Representative: Petraz, Gilberto Luigi
(86) International application number: PCT/CN2010/070546
(87) International publication number: WO 2010/118651

(56) References cited:
- CN-A- 1 981 712
- CN-Y- 2 555 778
- CN-Y- 2 684 770
- RU-C1- 2 129 413
- US-A- 5 330 488
- US-A- 5 562 685
- US-A1- 2008 091 219

## Description

### FIELD OF THE INVENTION

The present invention relates to a field of medical treatment device, and more particularly refers to a suture fixation apparatus of the temporomandibular joint (TMJ) disc.

### BACKGROUND OF THE INVENTION

Since the 1990s, there has been an attempt by some surgeons to use minimally invasive endoscopic surgery to cure the anteriorly displaced temporomandibular joint (TMJ) disc. What all these techniques have in common is that the TMJ disc is pulled back to its normal position by using a traction suture through the posterior band of the TMJ disc. However, post-operative imaging shows that most of the discs had not been repositioned. Since the 1990s, the present inventor followed the techniques advocated by surgeons in the U.S. (McCain, 1992) and Japan (Ohnishi, 1991) but more than 90% of the TMJ discs could not be repositioned to normal position.

US 5,562,685 disclosed a surgical instrument for placing suture or fasteners. The surgical instrument is comprised of an elongated handle having a coiled projection at its distal end, wherein the coiled projection is employed in penetrating and positioning a length of suture or fastener in tissue, for example, as in tissue proximation. The instrument is a needle and inside it a core is inserted having a wire snare welded to its distal end.

US 5,330,488 disclosed a surgically implanted catheter, wherein the surgically implanted catheter is sealed at its proximal end with a material which may be repeatedly punctured by a needle and which reseals when the needle is removed.

US 2008/091219 disclosed an apparatus for suturing tissue, wherein the apparatus comprises: a housing; a first needle mounted to the housing; a second needle mounted to the housing; a suture having a leading portion and a trailing portion; a first structure associated with the first needle for passing the leading portion of the suture from a near side of the tissue to a far side of the tissue; and a second structure associated with the second needle for retracting the leading portion of the suture from the far side of the tissue back to the near side of the tissue.

However, some of the disadvantages of these techniques include the necessity of using multiple cannulas for introducing additional surgical instruments such as forceps, cutting instruments and light sources.

This drove the inventers to reconsider the surgery and to examine the drawbacks of the surgical techniques which are mostly represented as: (1) the direction of the traction suture was not in the same direction as the displaced TMJ disc; and (2) only one suture was used for the disc repositioning, and the suture was positioned only in the lateral 1/3 of the disc, making it impossible to stabilize the entire disc which in mediolateral dimension is 2 cm wide. In order to conquer the drawbacks, the present inventor prepared a suture apparatus which is applicable to the joint disc restoration fixing surgery under the new joint mirror.

Another problem is that there is lack of appropriate suturing devices for arthroscopic TMJ disc repositioning, so that the repositioning surgery has not been developed well. For example, it is hard for the currently-used calculi forceps to catch the suture and pull it out through the skin. During surgery, the suture is very slippery, which causes the surgeon to repeat one same action and increasing patient's pain. Second, the suture needle has no handles which makes it hard to operate.

### SUMMARY OF THE INVENTION

An object of the present invention is to provide a suture device which can help reposition and stabilize the displaced TMJ disc back to normal position by arthrosopic surgery. The device is easy to operate and less likely to slip during surgery.

Therefore, the technical solution of the present invention is disclosed as follows. A suture fixation apparatus of the temporomandibular joint (TMJ) disc, characterized in that: comprising a first suture needle and a first inner core, a second suture needle and a second inner core and a third suture needle, wherein,
The proximal end of the first suture needle is connected to a big handle having an axis by a socket, a vertical groove lying in a plane comprising said axis extend into big handle from its surface to said axis, a horizontal groove of the same width as the vertical groove and lying in a plane perpendicular to said axis extend into the vertical groove from its surface to said axis in the end of the big handle near the socket, the first inner core comprises a seamless shaft having an outer diameter less than the inner diameter of the first suture needle, a wire snare is welded at both its ends to the distal end of the seamless shaft, a 90° bend is made near the proximal end of seamless shaft, a small handle smaller than the big handle is connected at the proximal end of the seamless shaft, and the first inner core can be inserted into the vertical groove and into the first suture needle until the wire snare is exposed at the distal end of the first suture needle, at which time the 90° bend of the seamless shaft is able to lock in either the left or right part of the horizontal groove; the proximal end of the second suture needle is connected to another big handle having an axis by a socket, a vertical groove lying in a plane comprising said axis extend into the big handle from its surface to said axis, a horizontal groove of the same width as the vertical groove and lying in a plane perpendicular to said axis extends into the big handle from its surface to said axis in the end of the big handle near the socket, the second inner core comprises a seamless shaft having an outer diameter less than the inner diameter of second suture needle, a crochet hook having the same inner diameter as the seamless shaft is welded at the distal end of the seamless shaft, a 90° bend is made near the proximal end of the seamless shaft, a small handle smaller that the big handle is connected at the proximal end of the seamless shaft, the second inner core can be inserted into the vertical groove and into the second suture needle until the crochet hook is exposed at the distal end of the second suture needle, at which time the 90° bend of the seamless shaft is able to lock in either the left or right part of the horizontal groove; and the third suture needle is a normal No. 12 puncture needle.

The wire snare includes two bulges. The width of the distal end of the wire snare is smaller than the outer diameter of the seamless shaft, and the inner diameter of the proximal end of the wire snare is greater than the outer diameter of the seamless shaft. The inside of the hook of the crochet hook can exactly fit one seam.

The suture thread is made of 2-0 polyester knitting fiber. The thread comprises at least two zones: a guiding zone and a functional zone. The guiding zone is located at one or two ends of the thread and is strengthened by adhesion agent. The functional zone is soft without adhesion agent. The guiding zone and the functional zone are separated by different colors.

The present invention can also include a small hole in the middle of the small handle on the proximal end of the first and second inner core.

The present invention has the following benefits:
The big handles of the first and second suture needles are convenient to operate during surgery. The horizontal groove on it which is perpendicular to the circular channel can lock the inner core apropos. The wire snare on top of the first inner core, which replaces the former calculi forceps, makes it easy to completely capture the thread with the suture needle, so that the thread is less likely to slip during the surgery. And the crochet hook on top of the second inner core can hold the thread apropos, thereby locking the thread between the crochet hook and the suture needle tightly in order to pull the thread out without losing it during operation. The present invention makes the surgery successful on the first attempt, rather than repeating one action again and again, which significantly reduces the trauma to the patient.

### DESCRIPTION OF THE DRAWINGS

Accompanying with the drawings of the description, a further detailed description to the invention is described below.
Fig. 1A is the first suture needle, Fig. 1B is the first inner core, and Fig. 1C is the combination of the first suture needle and the first inner core according to one embodiment of the present invention;
Fig. 2A is the second suture needle, Fig. 2B is the second inner core, and Fig. 2C is the combination of the second suture needle and the second inner core;
Fig. 3 is a schematic view of the third suture needle according to one embodiment of the present invention;
Fig. 4 is a schematic enlarged view of the wire snare on top of the first inner core according to one embodiment of the present invention; and
Fig. 5 is a schematic enlarged view of the crochet hook on top of the second inner core according to one embodiment of the present invention.

### DETAILED DESCRIPTION OF THE PREFERRED EMBODIMENTS

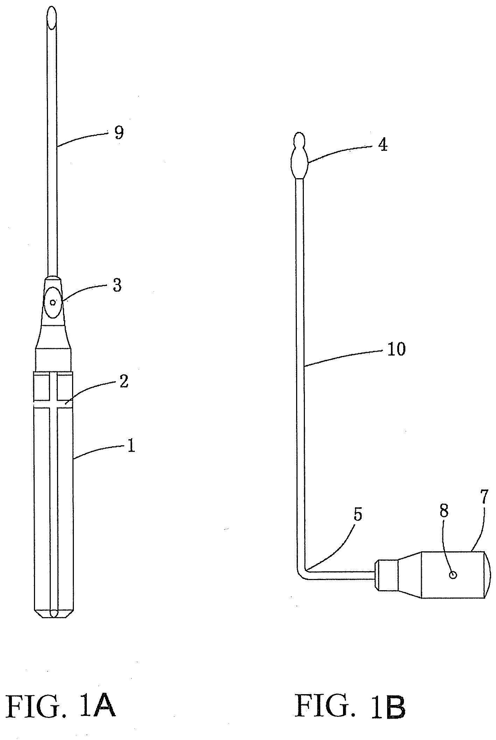

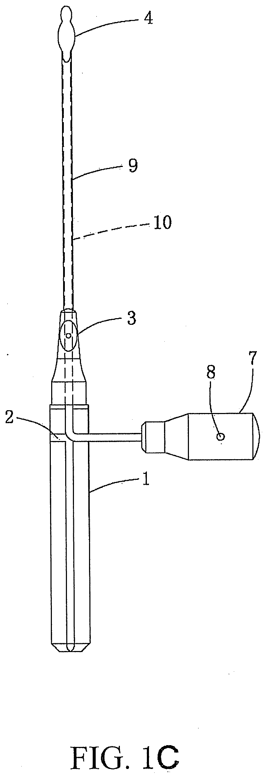

Fig. 1A is the first suture needle, which is modified from No. 12 puncture needle. The proximal end of the suture needle 9is connected the big handle 1 having an axis by a socket 3.Avertical groove lying in a plane comprising said axis extends into the big handle 1 from its surface to said axis. A horizontal groove 2 of the same width as the vertical groove and lying in a plane is formed perpendicular to said axis extends into the big handle 1 from its surface to the axis in the end of the big handle 1 near the socket 3.Fig. 1B is the first inner core, including a seamless shaft 10 having an outer diameter less than the inner diameter of the first suture needle 9. A two-bulge wire snare 4 is welded at both its ends to the distal end of the seamless shaft 10. A 90° bend 5 is made near the proximal end of the seamless shaft 10. A small handle 7 is connected at the proximal end of the seamless shaft 10. Fig. 1C is the combination of the first suture needle and the first inner core. The first inner core could pass through the vertical groove in the big handle 1 of the first suture needle. And the 90° bend is able to lock in either the left or right part of the horizontal groove 2, so that the wire snare 4 on top of it can come out from the top of the first suture needle 9.

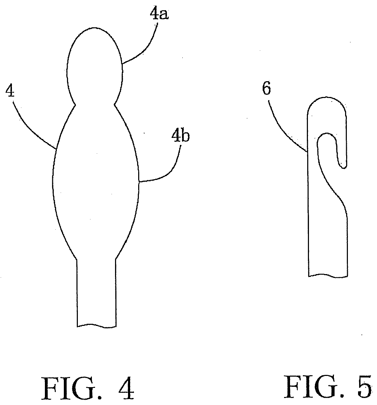

Fig. 4 is the enlargement view of the wire snare 4 which includes two bulges. The diameter of the first bulge 4a is smaller than the outer diameter of the seamless shaft, and the diameter of the second bulge 4b is greater than the outer diameter of the seamless shaft. The entire wire snare 4 is elastic. It is convenient for the first bulge 4a with a smaller diameter to pass through the circular channel from the bottom of the big handle 1, and the second bulge 4b with a greater diameter to pass through the suture during a surgery.

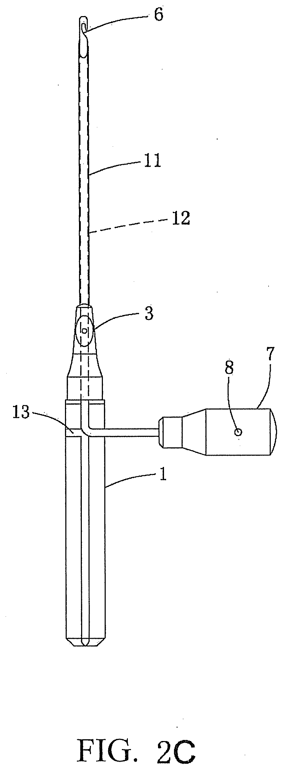

Fig. 2A is the second suture needle which is also modified from No. 12 puncture needle. The proximal end of the suture needle 11 is connected to the big handle 1 having an axis by a socket 3. A vertical groove lying in a plane comprising said axis extends into the big handle 1 from its surface to said axis. A horizontal groove 13 of the same width as the vertical groove and lying in a plane perpendicular to said axis extends into the big handle 1 from its surface to the axis in the end of the big handle 1 near the socket 3. Unlike the first suture needle, the diameter of the second suture needle 11 is a little bit smaller than the first suture needle 9, and so as the vertical and horizontal grooves of those suture needles, in order to fit the different diameter of the inner core (Fig. 2B) combined with the suture needles. Fig. 2B is the second inner core, including a seamless shaft 12 having an outer diameter less than the inner diameter of the second suture needle (11). A crochet hook having the same outer diameter as the seamless shaft (12) is welded at the distal end of the seamless shaft 12. A 90° bend 5 is made near the proximal end of the seamless shaft 12.A small handle 7 is connected at the proximal end of the seamless shaft 12. Fig. 2C is the combination of the second suture needle and the second inner core. The second inner core could pass through the vertical groove 13 of the big handle 1 of the second suture needle. The 90° bend is able to lock in either the left or right part of the horizontal groove 13, so that the crochet hook 6 can expose from the top of the first suture needle 11.

Fig. 5 is the enlargement view of the crochet hook 6, which can pass through only one suture thread.

The suture thread is made of 2-0 polyester knitting fiber. The thread comprises at least two zones: a guiding zone and a functional zone. The guiding zone is located at one or two ends of the thread and is strengthened by adhesion agent. The functional zone is soft without adhesion agent. The guiding zone and the functional zone are separated by different colors.

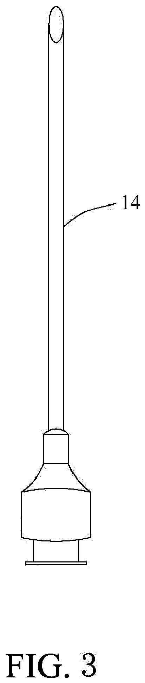

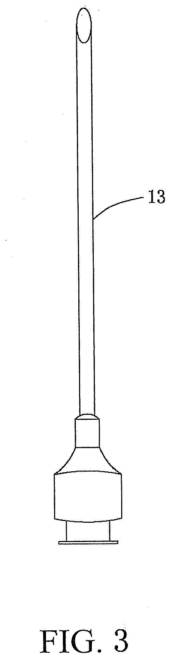

Fig. 3 is the third suture needle 14, and is also made from No. 12 puncture needle.

As one of the preferable embodiments of the present invention, a hole 8 could be in the small handle 7 to strengthen the safety of the present invention. A thread passing through the hole can be tied on the doctor's hand during operation to prevent accidental slip of the suture. A hole through which insertion of the inner core can also be viewed is also in the socket 3,to help inspecting the entering situation of the inner core.

Some humanity details can also be added into the present invention. For example, the surface of the big handle 1 could be made roughness for preventing slipper of the device during operation; in order to use conveniently, the first suture needle and the first inner core, the second suture needle and the second inner core can be painted in the same color when choosing for combined use during operation.
The device can be used as followed during operation: (1) First, using the third suture needle 14 (Fig.3) to puncture into the upper joint space at the top of the condyle. Let the assistant hold the arthroscope, and the operator continues to puncture with the third suture needle 14 into the TMJ disc at the lateral 1/3 point of the junction between the disc and the posterior band. The third suture needle 14 should puncture firstly going downward into the lower joint space, then going inside to the point of medial 1/3 of the junction and upward through the disc and out into the upper joint space again; (2) Let the patient keep mouth open, one hand of the assistant holds the arthroscope to keep stable vision, the other hand of the assistant holds the third suture needle 14 (Fig. 3) upward against the back of the condyle to stabilize the joint disc; (3) The operator inserts the first suture needle (Fig. 1A) and the first inner core (Fig. 1B) from the patient's external auditory canal. Be careful not to puncture the cartilage by the first suture needle. Then the operator put the hard part of the special thread into the third suture needle 14, adjust the position of the needle pinpoint to let the thread pass through the wire snare 4 of the first inner core (Fig. 1B). The thread was locked by turning the small handle of the first inner core into either right or left side to string up the thread and was pulled out of the first suture needle (Fig. 1A) from the external auditory canal. The half needle suture is done; (4) the operator pulls the third suture needle 14 (Fig 3) with the rest of the thread back to the first puncture point of the joint disc, and stays in the upper joint space, then adjusts the length of the thread. The assistant helps the third suture needle 14 (Fig. 3) to press the joint disc forward and downward, so that it is convenient for the operator to put the second suture needle (Fig. 2A) through the patient's external auditory canal. The crochet hook 6 on top of the second inner core (Fig. 2B) hooks the thread, and pulls it out of the external auditory canal. The first suture of disc repositioning is finished; (5) check the position of the joint disc under the arthrosope to see if it is repositioned and to find out whether a second suture is needed and the position of the suture.

## Claims

1. A suture fixation apparatus of the temporomandibular joint (TMJ) disc comprising a first suture needle (9) and a first inner core, a second suture needle (11) and a second inner core and a third suture needle (14), wherein,
a proximal end of the first suture needle (9) is connected to a big handle (1) having an axis by a socket (3), a vertical groove lying in a plane comprising said axis extends into the big handle (1) from its surface to said axis, a horizontal groove (2) of the same width as the vertical groove and lying in a plane perpendicular to said axis extends into the big handle (1) from its surface to the axis in the end of the big handle (1) near the socket (3), the first inner core comprises a seamless shaft (10) having an outer diameter less than the inner diameter of the first suture needle (9), a wire snare (4) is welded at both its ends to the distal end of the seamless shaft (10), a 90° bend is made near the proximal end of the seamless shaft (10), a small handle (7) smaller that the big handle (1) is connected at the proximal end of the seamless shaft (10), and the first inner core can be inserted into the vertical groove and into the first suture needle (9) until the wire snare (4) is exposed at the distal end of the first suture needle (9), at which time the 90° bend of the seamless shaft (10) is able to lock in either the left or right part of the horizontal groove (2);
a proximal end of the second suture needle (11) is connected to another big handle (1) having an axis by a socket (3), a vertical groove (13) lying in a plane comprising said axis extends into the big handle (1) from its surface to said axis, a horizontal groove of the same width as the vertical groove and lying in a plane perpendicular to said axis extends into the big handle (1) from its surface to the axis in the end of the big handle (1) near the socket (3), the second inner core comprises a seamless shaft (12) having an outer diameter less than the inner diameter of the second suture needle (11), a crochet hook (6) having the same outer diameter as the seamless shaft (12) is welded at the distal end of the seamless shaft (12), a 90° bend is made near the proximal end of the seamless shaft (12), a small handle (7) smaller that the big handle (1) is connected at the proximal end of the seamless shaft (12), and the second inner core can be inserted into the vertical groove and into the second suture needle (11) until the crochet hook (6) is exposed at the distal end of the second suture needle (11), at which time the 90° bend of the seamless shaft (12) is able to lock in either the left or right part of the horizontal groove (13); and
the third suture needle (14) is a normal No. 12 puncture needle.

2. The suture fixation apparatus of the temporomandibular joint (TMJ) disc according to claim 1, **characterized in that**: the width of the proximal end of the wire snare (4) is greater than the outer diameter of the seamless shaft (10), and the width of the distal end of the wire snare (4) is less than the outer diameter of the seamless shaft (10).

3. The suture fixation apparatus of the TMJ disc according to claim 1, **characterized in that**: the inside of the hook of the crochet hook (6) can exactly fit one suture.

4. The suture fixation apparatus of the TMJ disc according to claim 1, **characterized in that**: there is a hole (8) in the middle of each small handle (7).

5. The suture fixation apparatus of the TMJ disc according to claim 1, **characterized in that**: there is a monitoring hole is in the middle of the each socket (3).

6. The suture fixation apparatus of the TMJ disc according to claim 1, **characterized in that**: the big handle (1) and the small handle (7) are predetermined roughened.

7. The suture fixation apparatus of the TMJ disc according to claim 1, **characterized in that**: the colors of the big handle (1) of the first suture needle (9) and the small handle (7) of the first inner core are the same, and the colors of the big handle (1) of the second suture needle (11) and the small handle of the second inner core are the same.

8. The suture fixation apparatus of the TMJ disc according to claim 1, **characterized in that**: the inner diameter of the first suture needle (9) is slightly greater than the inner diameter of the second suture needle (11).

## Patentansprüche

1. Nahtfixierungsvorrichtung der Kiefergelenkscheibe, umfassend eine erste Nahtnadel (9) und einen ersten inneren Kern, eine zweite Nahtnadel (11) und einen zweiten inneren Kern und eine dritte Nahtnadel (14), worin
ein proximales Ende der ersten Nahtnadel (9) mit einem großen Handgriff (1) verbunden ist, der eine Achse an einer Buchse (3) hat, eine vertikale Nut, die in einer Ebene liegt, die diese Achse umfasst, sich in den großen Handgriff (1) hinein von dessen Oberfläche bis zu der genannten Achse erstreckt, eine horizontale Nut (2), die dieselbe Breite wie die vertikale Nut hat und in einer Ebene liegt, die zur genannten Achse senkrecht ist, sich in den großen Handgriff (1) hinein von dessen Oberfläche bis zur Achse am Ende des großen Handgriffs (1) neben der Buchse (3) erstreckt, der erste innere Kern einen nahtlosen Schaft (10) umfasst, der einen Außendurchmesser hat, der kleiner als der Innendurchmesser der ersten Nahtnadel (9) ist, eine Drahtschlinge (4) an ihren beiden Enden mit dem distalen Ende des nahtlosen Schaftes (10) geschweißt ist, eine 90°-Biegung neben dem proximalen Ende des nahtlosen Schaftes (10) erzeugt wird, ein kleiner Handgriff (7), der kleiner als der große Handgriff (1) ist, am proximalen Ende des nahtlosen Schaftes (10) verbunden ist, und der erste innere Kern in die vertikale Nut und in die erste Nahtnadel (9) hinein so weit eingefügt werden kann, bis die Drahtschlinge (4) am distalen Ende der ersten Nahtnadel (9) offenliegend ist, an welchem Zeitpunkt die 90°-Biegung des nahtlosen Schaftes (10) imstande ist, entweder in den linken oder in den rechten Teil der horizontalen Nut (2) einzurasten;
ein proximales Ende der zweiten Nahtnadel (11) mit einem anderen großen Handgriff (1) verbunden ist, der eine Achse an einer Buchse (3) hat, eine vertikale Nut (13), die in einer Ebene liegt, die diese Achse umfasst, sich in den großen Handgriff (1) hinein von dessen Oberfläche bis zu der genannten Achse erstreckt, eine horizontale Nut, die dieselbe Breite wie die vertikale Nut hat und in einer Ebene liegt, die zur genannten Achse senkrecht ist, sich in den großen Handgriff (1) hinein von dessen Oberfläche bis zur Achse am Ende des großen Handgriffs (1) neben der Buchse (3) erstreckt, der zweite innere Kern einen nahtlosen Schaft (12) umfasst, der einen Außendurchmesser hat, der kleiner als der Innendurchmesser der zweiten Nahtnadel (11) ist, ein Häkelhaken (6), der den gleichen Außendurchmesser wie der nahtlose Schaft (12) hat, am distalen Ende des nahtlosen Schaftes (12) geschweißt ist, eine 90°-Biegung neben dem proximalen Ende des nahtlosen Schaftes (12) erzeugt wird, ein kleiner Handgriff (7), der kleiner als der große Handgriff (1) ist, am proximalen Ende des nahtlosen Schaftes (12) verbunden ist, und der zweite innere Kern in die vertikale Nut und in die zweite Nahtnadel (11) hinein so weit eingefügt werden kann, bis der Häkelhaken (6) am distalen Ende der zweiten Nahtnadel (11) offenliegend ist, an welchem Zeitpunkt die 90°-Biegung des nahtlosen Schaftes (12) imstande ist, entweder in den linken oder in den rechten Teil der horizontalen Nut (13) einzurasten; und
die dritte Nahtnadel (14) eine übliche Punktionsnadel Nr. 12 ist.

2. Nahtfixierungsvorrichtung der Kiefergelenkscheibe nach Anspruch 1, **dadurch gekennzeichnet, dass**: die Breite des proximalen Endes der Drahtschlinge (4) größer als der Außendurchmesser des nahtlosen Schaftes (10) ist, und die Breite des distalen Endes der Drahtschlinge (4) kleiner als der Außendurchmesser des nahtlosen Schaftes (10) ist.

3. Nahtfixierungsvorrichtung der Kiefergelenkscheibe nach Anspruch 1, **dadurch gekennzeichnet, dass**: das Innere des Hakens des Häkelhakens (6) genau zu einer Naht passen kann.

4. Nahtfixierungsvorrichtung der Kiefergelenkscheibe nach Anspruch 1, **dadurch gekennzeichnet, dass**: es ein Loch (8) in der Mitte jedes kleinen Handgriffs (7) gibt.

5. Nahtfixierungsvorrichtung der Kiefergelenkscheibe nach Anspruch 1, **dadurch gekennzeichnet, dass**: es ein Überwachungsloch (8) in der Mitte jeder Buchse (3) gibt.

6. Nahtfixierungsvorrichtung der Kiefergelenkscheibe nach Anspruch 1, **dadurch gekennzeichnet, dass**: der große Handgriff (1) und der kleine Handgriff (7) vorbestimmt angeraut sind.

7. Nahtfixierungsvorrichtung der Kiefergelenkscheibe nach Anspruch 1, **dadurch gekennzeichnet, dass**: die Farben des großen Handgriffs (1) der ersten Nahtnadel (9) und des kleinen Handgriffs (7) des ersten inneren Kerns gleich sind, und die Farben des großen Handgriffs (1) der zweiten Nahtnadel (11) und des kleinen Handgriffs des zweiten inneren Kerns gleich sind.

8. Nahtfixierungsvorrichtung der Kiefergelenkscheibe nach Anspruch 1, **dadurch gekennzeichnet, dass**: der Innendurchmesser der ersten Nahtnadel (9) etwas größer als der Innendurchmesser der zweiten Nahtnadel (11) ist.

## Revendications

1. Appareil de fixation de sutures du disque de l'articulation temporo-mandibulaire (ATM) comprenant une première aiguille à suture (9) et un premier noyau interne, une deuxième aiguille à suture (11) et un deuxième noyau interne et une troisième aiguille à suture (14), dans lequel
une extrémité proximale de la première aiguille à suture (9) est reliée à une grande poignée (1) s'étendant le long d'un axe, à l'aide d'une douille (3), une rainure verticale située dans un plan comprenant ledit axe, s'étend dans la grande poignée (1) de sa surface audit axe, une rainure horizontale de la même largeur que la rainure verticale, située dans un plan perpendiculaire audit axe s'étend dans la grande poignée de sa surface à l'axe dans l'extrémité de la grande poignée (1) près de la douille (3), le premier noyau interne comprend un arbre sans soudure (10) présentant un diamètre externe inférieur au diamètre interne de la première aiguille à suture (9), une boucle en fil métallique est soudé à ses extrémités à l'extrémité distale de l'arbre sans soudure (10), un coude à 90° est formée près de l'extrémité proximale (10), une petite poignée (7) plus petite que la grande poignée (1) est reliée à l'extrémité proximale de l'arbre sans soudure (10), et le premier noyau interne peut s'insérer dans la rainure verticale et dans la première aiguille à suture (9) jusqu'à ce que la boucle en fil métallique ne soit exposé à l'extrémité distale de la première aiguille à suture (9), ce qui permet le blocage du coude à 90° de l'arbre sans soudure (10) dans la partie gauche ou droite de la rainure horizontale (2) ;
une extrémité proximale de la deuxième aiguille à suture (11) est relié à une autre grande poignée (1) s'étendant le long d'un axe, à l'aide d'une douille (3), une rainure verticale (13) située dans un plan comprenant ledit axe s'étend dans la grande poignée (1) de sa surface audit axe, une rainure horizontale de la même largeur que la rainure verticale et située dans un plan perpendiculaire audit axe s'étend dans la grande poignée (1) de sa surface à l'axe dans l'extrémité de la grande poignée (1) près de la douille (3), le deuxième noyau interne comprend un arbre sans soudure (12) ayant un diamètre extérieur inférieur au diamètre intérieur de la deuxième aiguille à suture (11), un crochet (6) présentant le même diamètre externe que l'arbre sans soudure (12) est soudé à l'extrémité distale de l'arbre sans soudure (12), un coude à 90° est formé près de l'extrémité proximale de l'arbre sans soudure (12), une petite poignée (7) plus petite que la grande poignée (1) est reliée à l'extrémité proximale de l'arbre sans soudure (12), et le deuxième noyau interne peut s'insérer dans la rainure verticale et dans la deuxième aiguille à suture (11) jusqu'à ce que le crochet (6) ne soit exposé à l'extrémité distale de la deuxième aiguille à suture (11), ce qui permet le blocage du coude à 90° de l'arbre sans soudure (12) es dans la partie gauche ou droite de la rainure horizontale (13); et la troisième aiguille à suture (14) est une aiguille de ponction no. 12.

2. Appareil de fixation de sutures du disque de l'articulation temporo-mandibulaire (ATM) selon la revendication 1, **caractérisé en ce que** : la largeur de l'extrémité proximale de la boucle en fil métallique (4) est supérieure au diamètre externe de l'arbre sans soudure (10), et la largeur de l'extrémité distale de la boucle en fil métallique (4) est inférieure au diamètre externe de l'arbre sans soudure (10).

3. Appareil de fixation de sutures du disque de l'ATM selon la revendication 1, **caractérisé en ce que** : l'intérieur due la cheville du crochet (6) peut correspondre exactement à une suture.

4. Appareil de fixation de sutures du disque de l'ATM selon la revendication 1, **caractérisé en ce que** : au milieu de chaque petite poignée (7) il y a un trou (8).

5. Appareil de fixation de sutures du disque de l'ATM selon la revendication 1, **caractérisé en ce que** : au milieu de chaque douille il y a un trou d'inspection.

6. Appareil de fixation de sutures du disque de l'ATM selon la revendication 1, **caractérisé en ce que** : la grande poignée (1) et la petite poignée (7) ont une rugosité prédéterminée.

7. Appareil de fixation de sutures du disque de l'ATM selon la revendication 1, **caractérisé en ce que** : les couleurs de la grande poignée (1) de la première aiguille à suture (9) et de la petite poignée (7) du premier noyau interne sont les mêmes, et les couleurs de la grande poignée (1) de la deuxième aiguille à suture (11) et de la petite poignée du deuxième noyau interne sont les mêmes.

8. Appareil de fixation de sutures du disque de l'ATM selon la revendication 1, **caractérisé en ce que** : le diamètre interne de la première aiguille à suture (9) est légèrement supérieur au diamètre interne de la deuxième aiguille à suture (11) .
